# EUROPEAN PATENT APPLICATION

(11) **EP 4 534 074 A1**
(43) Date of publication of application: **09.04.2025**
(21) Application number: 23201668.3
(22) Date of filing: 04.10.2023
(51) Int. Cl.: A61K 9/08, A61K 47/10, A61K 38/09, A61K 47/12, A61P 15/08

(54) **LIQUID CETRORELIX COMPOSITION**

(71) Applicant: Ares Trading S.A., 1170 Aubonne (CH)
(72) Inventor: RINALDI, Gianluca, 00012 Guidonia Montecelio RM (IT); PISTACCHIO, Alessandra, 00012 Guidonia Montecelio RM (IT); LICARI, Giuseppe, 1804 Corsier-sur-Vevey (CH); POLITI, Jane, 00012 Guidonia Montecelio RM (IT); LAURITZEN, Alain, 1804 Corsier-sur-Vevey (CH); AMIDI, Maryam, 00012 Guidonia Montecelio RM (IT)
(74) Representative: Merck Serono S.A. Intellectual Property

(57) **Abstract**

The present invention refers to a liquid pharmaceutical Cetrorelix composition comprising a stabilizing agent selected from poloxamer having an t average molecular weight of 7,000-12,000 g/mol and/or benzyl alcohol, a pharmaceutical dosage form comprising said liquid pharmaceutical composition, and their use in inhibiting premature luteinizing hormone surges in woman undergoing controlled ovarian stimulation. Further, a method for preparing the liquid pharmaceutical Cetrorelix composition and pharmaceutical dosage form is disclosed.

## Description

The present invention refers to a liquid pharmaceutical Cetrorelix composition. The liquid pharmaceutical composition comprises and preferably consists of Cetrorelix (salt), a stabilizing agent selected from poloxamer having an average molecular weight of 7,000-12,000 g/mol, benzyl alcohol, or a mixture thereof, an osmotic agent, and a pharmaceutically acceptable solvent comprising an organic acid.

Moreover, the present invention refers to a pharmaceutical dosage form comprising the liquid pharmaceutical composition of the invention in a container, such as a vial or an injection device, e.g., a pre-filled syringe.

In a further aspect, the present invention refers to the liquid pharmaceutical composition or pharmaceutical dosage form according to the invention for use in inhibiting premature luteinizing hormone surges in woman undergoing controlled ovarian stimulation.

In still a further aspect, the present invention refers to a method for preparing the liquid pharmaceutical composition or pharmaceutical dosage form according to the invention, the method comprising the steps of providing a first composition comprising a stabilizing agent selected from poloxamer having an average molecular weight of 7,000-12,000 g/mol, benzyl alcohol, or a mixture thereof, and an osmotic agent, preferably D-mannitol; providing a second composition comprising or consisting of Cetrorelix (salt), preferably Cetrorelix acetate; mixing the first composition and the second composition to obtain a liquid composition, and optionally adding an organic acid to the mixture obtained, wherein a pharmaceutically acceptable solvent is present in the first composition and/or wherein a pharmaceutically acceptable solvent is present in the second composition.

Cetrorelix (N-acetyl-3-(2-naphthyl)-D-alanyl-4-chloro-D-phenylalanyl-3-(3-pyridyl)-D-alanyl-L-seryl-L-tyrosyl-D-citrullyl-L-leucyl-L-arginyl-L-prolyl-D-alaninamide) is a decapeptide with gonadotropin-releasing hormone (GnRH) antagonistic activity. It blocks the effects of luteinising-hormone releasing hormone (LHRH) in the body. LHRH controls the production and release of luteinising hormone (LH), which causes ovulation. During fertility treatment, ovarian stimulation is used to make the ovaries produce more egg cells. By blocking the effect of LHRH, Cetrorelix stops the production of LH, and therefore prevents premature ovulation, which can result in the release of egg cells that are immature and unsuitable for use in techniques such as in vitro fertilisation (IVF).

A pharmaceutically acceptable Cetrorelix salt, Cetrorelix acetate, is currently marketed in the form of a freeze-dried powder co-packed with water for injection to reconstitute the powder and to obtain a solution for injection (Cetrotide^{®}). Each powder vial contains 0.25 mg Cetrorelix acetate and mannitol. The pH of the reconstituted solution is 4.0-6.0. Due to stability reasons, the reconstituted solution is for immediate use only.

There is thus a need to provide a stable liquid composition comprising Cetrorelix, which can be provided as a ready-to-use pharmaceutical dosage form without the need of reconstitution prior to administration.

US 2013/0303464 A1 discloses a pharmaceutical preparation of Cetrorelix acetate, further comprising glacial acetic acid, a tonicity agent, and water. The pH of the preparation is adjusted to about 3.

The present inventors surprisingly found that liquid Cetrorelix compositions can be stabilized by the incorporation of particular stabilizing agents. It is therefore an object of the present invention to provide an improved liquid pharmaceutical preparation of Cetrorelix, having high stability and prolonged shelf life. Further, by providing stable liquid Cetrorelix compositions, the administration of Cetrorelix is more user friendly and the supply-chain complexity is reduced in comparison to solid Cetrorelix compositions.

One aspect of the present invention is a liquid pharmaceutical composition, comprising Cetrorelix or a pharmaceutically acceptable salt thereof, a stabilizing agent selected from poloxamer having an average molecular weight of 7,000-12,000 g/mol, benzyl alcohol, or a mixture thereof, an osmotic agent, and a pharmaceutically acceptable solvent comprising an organic acid.

The pharmaceutical composition comprises Cetrorelix or a pharmaceutically acceptable salt thereof as an active agent. The pharmaceutically acceptable Cetrorelix salt preferably is Cetrorelix acetate. The amount of Cetrorelix (salt) present in the pharmaceutical composition can be adapted dependent on the pharmaceutical requirements. In one embodiment, the liquid pharmaceutical composition comprises Cetrorelix or a pharmaceutically acceptable salt thereof in an amount of 0.10 - 0.50 mg/ml, such as 0.10 - 0.50 mg/ml Cetrorelix or 0.10 - 0.50 mg/ml Cetrorelix acetate. In a preferred embodiment, the liquid pharmaceutical composition comprises an amount of 0.2 - 0.3 mg/ml Cetrorelix pure peptide in its acetate salt form. The Cetrorelix amount may be 0.25 mg/ml pure peptide in its acetate salt form.

The pharmaceutical composition further comprises a stabilizing agent selected from poloxamer having an average molecular weight of 7,000-12,000 g/mol, benzyl alcohol, or a mixture thereof. In one embodiment, the pharmaceutical composition comprises poloxamer having an average molecular weight of 7,000-12,000 g/mol as a stabilizing agent, preferably poloxamer having an average molecular weight of 7,500-10,000 g/mol. Poloxamers are non-ionic triblock copolymers composed of a central hydrophobic chain of polyoxypropylene (polypropylene oxide)) flanked by two hydrophilic chains of polyoxyethylene (poly(ethylene oxide)). A suitable poloxamer stabilizing agent according to the present invention is poloxamer 188 (having an average molecular weight of 7680-9510 g/mol). In another embodiment, the pharmaceutical composition comprises benzyl alcohol as a stabilizing agent.

Additional stabilizing agents are not required in the liquid Cetrorelix composition according to the present invention. Particularly, the liquid pharmaceutical composition according to the invention is preferably free of polysorbate and/or cyclodextrin.

The stabilizing agent may be comprised in the pharmaceutical composition in an amount of 0.5-20.0 mg/ml, preferably 1.0-15.0 mg/ml. The unit mg/ml as used herein defines the amount of a compound in mg relative to the total volume of the pharmaceutical composition in ml. For example, the stabilizing agent may be poloxamer 188 comprised in an amount of 0.5-10.0 mgl/ml, preferably 1.0-5.0 mg/ml, or benzyl alcohol comprised in an amount of 5.0-20.0 mg/ml, preferably 7.5-15.0 mg/ml.

The stabilizing agent serves to improve the stability of the liquid pharmaceutical composition according to the invention. It was surprisingly found that the present stabilizing agents allow for the provision of a particularly stable liquid Cetrorelix composition. Specifically, the presence of aggregates, particles and impurities is reduced or even avoided. By this means, relevant pharmaceutical properties such as pH, Cetrorelix protein content, and osmolality are maintained during storage. Even when subjected to external stress such as mechanical or thermal impact, the liquid pharmaceutical Cetrorelix composition according to the present invention exhibits desirable pharmaceutical properties and stability.

The liquid pharmaceutical composition further comprises an osmotic agent. Suitable osmotic agents may be selected from the group consisting of mannitol, glycerol, sorbitol, dextrose, sucrose, trehalose, or a combination thereof, preferably D-mannitol, sorbitol, or trehalose. In a particularly preferred embodiment, the osmotic agent is D-mannitol. The osmotic agent may be comprised in the pharmaceutical composition in an amount of 30.0-60.0 mg/ml, preferably 35.0-55.0 mg/ml.

The osmotic agent serves to adapt the osmolality of the liquid pharmaceutical composition according to the invention. The osmolality of the pharmaceutical composition may be 250-375 mOsm/kg, preferably 300-350 mOsm/kg. Specifically, the osmolality may be adapted according to the desired route of administration.

In a preferred embodiment, the pharmaceutical composition is for parenteral administration. Parenteral administration comprises intravenous, intramuscular, subcutaneous, and intradermal administration, such as injection and infusion, preferably subcutaneous injection. A suitable osmolality for parenteral pharmaceutical compositions is 250-375 mOsm/kg, preferably 300-350 mOsm/kg.

The liquid pharmaceutical composition further comprises a pharmaceutically acceptable solvent comprising an organic acid. The organic acid may be any suitable organic acid and is preferably selected from acetic acid, lactic acid, glutamic acid, gluconic acid, succinic acid, and a mixture thereof, more preferably acetic acid. The pharmaceutically acceptable solvent may comprise the organic acid in the form of an acidic buffer or a free organic acid. Additionally, the solvent may comprise water, such as water for injection. In a preferred embodiment, the pharmaceutically acceptable solvent is an aqueous organic acid or an aqueous acidic buffer, preferably an aqueous organic acid.

An acidic buffer according to the present invention comprises the organic acid and a salt thereof (in dissolved form). Suitable salts are metal salts, such as alkali metal salts, e.g., sodium salts and potassium salts, or alkaline earth metal salts, e.g., calcium salts, ammonium or amine salts. For example, the pharmaceutically acceptable solvent may comprise an acetate buffer, such as sodium acetate/acetic acid buffer or ammonium acetate/acetic acid buffer. The concentration of the acidic buffer, preferably acetate buffer, in the liquid pharmaceutical composition may be 20 mM.

In a preferred embodiment, the pharmaceutically acceptable solvent comprises the organic acid in the form of a free organic acid. The term "free organic acid" according to the present invention refers to an organic acid in the absence of a salt thereof. In a preferred embodiment, the pharmaceutically acceptable solvent of the invention is an aqueous free organic acid, preferably aqueous acetic acid. In the presence of water, the free organic acid is present in its protonated and deprotonated form.

The free organic acid may be selected from acetic acid, lactic acid, glutamic acid, gluconic acid, succinic acid, and a mixture thereof, preferably acetic acid, gluconic acid and glutamic acid. In a particularly preferred embodiment, the free organic acid is acetic acid. The present inventors surprisingly found that the stability of the liquid pharmaceutical composition can be further improved by the presence of free acetic acid comprised in the pharmaceutically acceptable solvent. In a preferred embodiment, the pharmaceutically acceptable solvent is aqueous acetic acid, e.g., comprised in a concentration of 0.05-0.50 mg/ml, preferably 0.10-0.30 mg/ml.

The organic acid may serve to adjust the pH of the liquid pharmaceutical composition. The pH of the pharmaceutical composition may be 4.0-6.0, preferably 4.3-5.1, and more preferably 4.5-5.0. The organic acid, preferably acetic acid, may thus be comprised in an amount to adjust the pH of the pharmaceutical composition to a value of 4.0-6.0, preferably 4.3-5.1, and more preferably 4.5-5.0.

The liquid pharmaceutical composition according to the invention may have any suitable form and is particularly in the form of a solution. In one embodiment, the liquid pharmaceutical composition is a single-dose formulation. A single-dose formulation particularly consists of Cetrorelix or a pharmaceutically acceptable salt thereof, a stabilizing agent selected from poloxamer having an average molecular weight of 7,000-12,000 g/mol, benzyl alcohol, or a mixture thereof, an osmotic agent, and a pharmaceutically acceptable solvent comprising an organic acid.

In another embodiment, the liquid pharmaceutical composition is a multi-dose formulation optionally comprising further excipients such as a pharmaceutically acceptable preservative in an effective amount. Pharmaceutically acceptable preservatives are known in the art and comprise m-cresol, phenol, benzylalcohol and benzalkonium chloride, for example. An "effective amount" of preservative means, that the amount is sufficient to prevent microbial growth in the liquid pharmaceutical composition. A multi-dose formulation particularly consists of Cetrorelix or a pharmaceutically acceptable salt thereof, a stabilizing agent selected from poloxamer having an average molecular weight of 7,000-12,000 g/mol, benzyl alcohol, or a mixture thereof, an osmotic agent, a pharmaceutically acceptable preservative in an effective amount, and a pharmaceutically acceptable solvent comprising an organic acid.

In a preferred embodiment, the present invention is directed to a liquid pharmaceutical composition comprising and preferably consisting of Cetrorelix acetate in a concentration of 0.10-0.50 mg/ml, poloxamer 188 in a concentration of 0.5-10.0 mg/ml, D-mannitol in a concentration of 35.0-60.0 mg/ml, acetic acid in a concentration to adjust the pH of the pharmaceutical composition to a value of 4.0-6.0 and water for injection. In a particularly preferred embodiment, the present invention is directed to a liquid pharmaceutical composition comprising and preferably consisting of Cetrorelix acetate in a concentration of 0.2 - 0.3 mg/ml Cetrorelix pure peptide in its acetate salt form, poloxamer 188 in a concentration of 1.5 mg/ml, D-mannitol in a concentration of 54.8 mg/ml, acetic acid in a concentration of 0.1 mg/ml and water for injection.

In a further preferred embodiment, the present invention is directed to a liquid pharmaceutical composition comprising and preferably consisting of Cetrorelix acetate in a concentration of 0.10-0.50 mg/ml, benzyl alcohol in a concentration of 5.0-20.0 mg/ml, D-mannitol in a concentration of 30.0-55.0 mg/ml, acetic acid in a concentration to adjust the pH of the pharmaceutical composition to a value of 4.0-6.0 and water for injection. In a particularly preferred embodiment, the present invention is directed to a liquid pharmaceutical composition comprising and preferably consisting of Cetrorelix acetate in a concentration of 0.2 - 0.3 mg/ml Cetrorelix pure peptide in its acetate salt form, benzyl alcohol in a concentration of 10 mg/ml, D-mannitol in a concentration of 37.8 mg/ml, acetic acid in a concentration of 0.1 mg/ml and water for injection.

In a further aspect, the present invention refers to a pharmaceutical dosage form comprising the liquid pharmaceutical composition according to the present invention as described herein in a container. Suitable containers for liquid pharmaceutical compositions are known to a person skilled in the art. The selection of a suitable container may depend on the desired route of administration. In a preferred embodiment, the pharmaceutical dosage form of the present invention is a parenteral dosage form, preferably for injection. A suitable container, particularly for a parenteral pharmaceutical dosage form, may be selected from an ampoule or vial such as a sterile vial, or an injection device such as a pre-filled syringe or an autoinjector.

The pharmaceutical dosage form may be a single-dose or multiple-dose dosage form. A single-dose dosage form may comprise the liquid pharmaceutical composition in a pre-filled syringe or an autoinjector, for example. A multiple-dose dosage form may comprise the liquid pharmaceutical composition in a sterile vial or a pre-filled cartridge, for example.

In a further aspect, the present invention refers to a liquid pharmaceutical composition or a pharmaceutical dosage form according to the present invention as described herein for use in inhibiting premature luteinizing hormone surges in woman undergoing controlled ovarian stimulation.

In a further aspect, the present invention refers to a method for inhibiting premature luteinizing hormone surges in woman undergoing controlled ovarian stimulation, comprising administering to the woman a pharmaceutically effective amount of a liquid pharmaceutical composition according to the present invention as described herein. The method may include the use of a pharmaceutical dosage form according to the present invention as described herein. In further aspects, a pharmaceutically effective amount of a liquid pharmaceutical composition according to the present invention may be administered to suppress serum estradiol levels in woman at risk of OHSS, post ovarian stimulation with gonadotropins and oocyte retrieval; or to down-regulate the pituitary pre-ovarian stimulation in post-pubertal/premenopausal females attempting to preserve their fertility options with medically assisted reproduction and oocytes/embryos cryopreservation prior to potential sterilizing effects of chemotherapeutic agents and pelvic radiation, or the need to surgically remove the gonads; or to random start controlled ovarian stimulation using an antagonist protocol, regardless of the phase of the menstrual cycle in post-pubertal/premenopausal females to preserve fertility options with medically assisted reproduction and cryopreservation oocytes or embryos prior to potential sterilizing effects of chemotherapeutic agents and pelvic radiation, or the need to surgically remove the gonads; or to provide protective effect on ovarian function, when used during chemotherapy in post-pubertal/premenopausal females; or to effect pituitary down-regulation in hormone controlled cryopreserved embryo/blastocyst replacement treatment cycle.

An effective amount of the liquid pharmaceutical composition can vary depending on the route of administration, the age and weight of the patient, the nature and severity of the diseases to be treated, and similar factors. The daily dose can be given as a single dose, which is to be administered once, or be subdivided into two or more daily doses.

The liquid pharmaceutical composition of the invention may be administered alone or as a combination therapy with further active agents.

In a still further aspect, the present invention refers to a method for preparing a liquid pharmaceutical composition or a pharmaceutical dosage form according to the present invention as described herein, the method comprising the steps of:
a) providing a first composition comprising a stabilizing agent selected from poloxamer having an average molecular weight of 7,000-12,000 g/mol, benzyl alcohol, or a mixture thereof, and an osmotic agent,
b) providing a second composition comprising or consisting of Cetrorelix or a pharmaceutically acceptable salt thereof,
c) mixing the first composition of step a) and the second composition of step b) to obtain a liquid composition, and
d) optionally adding an organic acid and/or a base such as sodium hydroxide to the mixture obtained in step c),
wherein a pharmaceutically acceptable solvent is present in the first composition and/or wherein a pharmaceutically acceptable solvent is present in the second composition.

In one embodiment, a pharmaceutically acceptable solvent is present in the first composition. In another embodiment, a pharmaceutically acceptable solvent is present in the second composition. In a preferred embodiment, a pharmaceutically acceptable solvent is present in the first composition and second composition. The solvent of the first and second composition may be the same or different. In one embodiment, the solvent of the first and second composition is the same and is preferably an aqueous organic acid, preferably acetic acid. In another embodiment, the solvent of the first and second composition is different, wherein preferably one of the solvents is water for injection and the other solvent is an aqueous organic acid, preferably aqueous acetic acid.

The first composition comprises the stabilizing agent, the osmotic agent and optionally a pharmaceutically acceptable solvent, such as aqueous acetic acid or water for injection. To obtain the first composition, step a) may comprise mixing the stabilizing agent and the osmotic agent. Optionally, step a) may comprise mixing the stabilizing agent, the osmotic agent and a pharmaceutically acceptable solvent, preferably aqueous acetic acid, to obtain the first composition.

The second composition comprises or consists of Cetrorelix or a pharmaceutically acceptable salt thereof, preferably Cetrorelix acetate, and optionally a pharmaceutically acceptable solvent, such as aqueous acetic acid or water for injection. To obtain the second composition, step b) may comprise mixing Cetrorelix or a pharmaceutically acceptable salt thereof, preferably Cetrorelix acetate, with a pharmaceutically acceptable solvent, preferably aqueous acetic acid. In one embodiment, the second composition is an aqueous solution of Cetrorelix or a pharmaceutically acceptable salt thereof, preferably Cetrorelix acetate. The aqueous Cetrorelix solution may further comprise an organic acid as described herein, preferably acetic acid.

In step c), the first composition and the second composition are mixed to obtain a liquid composition. Mixing may comprise adding the first composition and the second composition in a vessel and stirring. Further, step c) may comprises adding a pharmaceutically acceptable solvent, preferably water for injection, to the first and second composition. Thus, mixing of step c) may comprise adding the first composition, the second composition and the pharmaceutically acceptable solvent in a vessel and stirring.

In some embodiments, the method comprises a step d) of adding an organic acid and/or a base such as sodium hydroxide to the mixture obtained in step c). The organic acid is as described herein. Step d) is particularly performed in case at least one of the solvents of the first and second composition and specifically both solvents do not contain an organic acid.

If required, the method can further comprise a step of adjusting the pH of the liquid composition obtained in step c) or d) to a predetermined value by adding a pharmaceutically acceptable acid or base such as sodium hydroxide. The predetermined pH value can depend on the desired route of administration and may be 4.0-6.0, preferably 4.3-5.1, and more preferably 4.5-5.0. An adjustment of the pH may be performed by any pharmaceutically acceptable acid or base, such as acetic acid or sodium hydroxide.

The method of the invention may further comprise a step e) of sterile filtration of the liquid composition obtained in step c) or d) to obtain a sterile liquid composition. The liquid composition obtained in steps c) or d) or the sterile filtered, liquid composition obtained in step e) may then be filled in a container, such as an ampoule or vial e.g. a sterile vial, or an injection device, e.g. a syringe or an autoinjector, to obtain a pharmaceutical dosage form according to the present invention.

The invention is further defined by, but not limited to, the following items.
1. A liquid pharmaceutical composition, comprising or consisting of
   - Cetrorelix or a pharmaceutically acceptable salt thereof,
   - a stabilizing agent selected from poloxamer having an average molecular weight of 7,000-12,000 g/mol, benzyl alcohol, or a mixture thereof,
   - an osmotic agent, and
   - a pharmaceutically acceptable solvent comprising an organic acid.
2. The liquid pharmaceutical composition of item 1, wherein the pharmaceutically acceptable Cetrorelix salt is Cetrorelix acetate.
3. The liquid pharmaceutical composition according to any of the preceding items, comprising Cetrorelix or a pharmaceutically acceptable salt thereof in an amount of 0.10 - 0.50 mg/ml, preferably in an amount of 0.2 - 0.3 mg/ml Cetrorelix pure peptide in its acetate salt form.
4. The liquid pharmaceutical composition according to any of the preceding items, wherein the stabilizing agent comprises poloxamer having an average molecular weight of 7,500-10,000 g/mol.
5. The liquid pharmaceutical composition according to any of the preceding items, wherein the stabilizing agent is comprised in an amount of 0.5-20.0 mg/ml, preferably 1.0-15.0 mg/ml.
6. The liquid pharmaceutical composition according to any of the preceding items, wherein the stabilizing agent is poloxamer 188 comprised in an amount of 0.5-10.0 mg/ml, preferably 1.0-5.0 mg/ml.
7. The liquid pharmaceutical composition according to any of the preceding items, wherein the stabilizing agent is benzyl alcohol comprised in an amount of 5.0-20.0 mg/ml, preferably 7.5-15.0 mg/ml.
8. The liquid pharmaceutical composition according to any of the preceding items, wherein the osmotic agent is selected from the group consisting of mannitol, glycerol, sorbitol, sodium chloride, potassium chloride, dextrose, sucrose, trehalose, or a combination thereof, preferably D-mannitol.
9. The liquid pharmaceutical composition according to any of the preceding items, wherein the osmotic agent is comprised in an amount of 30.0-60.0 mg/ml, preferably 35.0-55.0 mg/ml.
10. The liquid pharmaceutical composition according to any of the preceding items, wherein the pharmaceutical composition has an osmolality of 250-375 mOsm/kg, preferably 300-350 mOsm/kg.
11. The liquid pharmaceutical composition according to any of the preceding items, wherein the pharmaceutically acceptable solvent comprises the organic acid in the form of an acidic buffer or a free organic acid, preferably wherein the pharmaceutically acceptable solvent is an aqueous organic acid or an aqueous acidic buffer.
12. The liquid pharmaceutical composition according to any of the preceding items, wherein the organic acid is acetic acid, lactic acid, glutamic acid, gluconic acid, succinic acid, or a mixture thereof, preferably acetic acid.
13. The liquid pharmaceutical composition according to any of the preceding items, wherein the acidic buffer is an acetate buffer, preferably comprised in a concentration of 20 mM.
14. The liquid pharmaceutical composition according to any of the preceding items, wherein the pharmaceutically acceptable solvent is an aqueous free organic acid, preferably aqueous acetic acid.
15. The liquid pharmaceutical composition according to any of the preceding items, wherein the pharmaceutically acceptable solvent is aqueous acetic acid comprised in an acetic acid concentration of 0.05-0.50 mg/ml, preferably 0.10-0.30 mg/ml.
16. The liquid pharmaceutical composition according to any of the preceding items, wherein the pH of the pharmaceutical composition is 4.0-6.0, preferably 4.3-5.1, and more preferably 4.5-5.0.
17. The liquid pharmaceutical composition according to any of the preceding items, wherein the liquid pharmaceutical composition is in the form of a solution.
18. The liquid pharmaceutical composition according to any of the preceding items, wherein the organic acid, preferably acetic acid, is comprised in an amount to adjust the pH of the pharmaceutical composition to a value of 4.0-6.0, preferably 4.3-5.1, and more preferably 4.5-5.0.
19. The liquid pharmaceutical composition according to any of the preceding items, wherein the pharmaceutical composition is for parenteral administration.
20. The liquid pharmaceutical composition according to any of the preceding items, wherein the pharmaceutical composition is free of polysorbate.
21. The liquid pharmaceutical composition according to any of the preceding items, wherein the pharmaceutical composition is free of cyclodextrin.
22. The liquid pharmaceutical composition according to any of the preceding items, wherein the pharmaceutical composition comprises and preferably consists of Cetrorelix acetate in a concentration of 0.2 - 0.3 mg/ml Cetrorelix pure peptide in its acetate salt form, poloxamer 188 in a concentration of 1.5 mg/ml, D-mannitol in a concentration of 54.8 mg/ml, acetic acid in a concentration of 0.1 mg/ml and water for injection.
23. The liquid pharmaceutical composition according to any of the preceding items, wherein the pharmaceutical composition comprises and preferably consists of Cetrorelix acetate in a concentration of 0.2 - 0.3 mg/ml Cetrorelix pure peptide in its acetate salt form, benzyl alcohol in a concentration of 10 mg/ml, D-mannitol in a concentration of 37.8 mg/ml, acetic acid in a concentration of 0.1 mg/ml and water for injection.
24. The liquid pharmaceutical composition according to any of the preceding items, which is a single-dose formulation.
24. The liquid pharmaceutical composition according to any of items 1-23, which is a multi-dose formulation comprising a pharmaceutically acceptable preservative in an effective amount.
25. A pharmaceutical dosage form comprising the liquid pharmaceutical composition according to any of items 1-24 in a container.
26. The pharmaceutical dosage form according to item 25, wherein the container is an ampoule or vial such as a sterile vial, an injection device such as a pre-filled syringe, or an autoinjector.
27. The pharmaceutical dosage form according to any of items 25-26, which is a parenteral dosage form, preferably for injection.
28. The pharmaceutical dosage form according to any of items 25-27, which is a single-dose or multiple-dose dosage form.
29. A liquid pharmaceutical composition according to any of items 1-24 or a pharmaceutical dosage form according to any of items 25-28 for use in inhibiting premature luteinizing hormone surges in woman undergoing controlled ovarian stimulation.
30. A method for preparing a liquid pharmaceutical composition according to any of items 1-24 or a pharmaceutical dosage form according to any of items 25-28, comprising the steps of:
   a) providing a first composition comprising a stabilizing agent selected from poloxamer having an average molecular weight of 7,000-12,000 g/mol, benzyl alcohol, or a mixture thereof, and an osmotic agent;
   b) providing a second composition comprising or consisting of Cetrorelix or a pharmaceutically acceptable salt thereof;
   c) mixing the first composition of step a) and the second composition of step b) to obtain a liquid composition; and
   d) optionally adding an organic acid to the mixture obtained in step c),
   wherein a pharmaceutically acceptable solvent is present in the first composition and/or wherein a pharmaceutically acceptable solvent is present in the second composition.
31. The method of item 30, wherein a pharmaceutically acceptable solvent is present in the first and second composition, wherein the solvent of the first and second composition is the same or different, preferably wherein one of the solvents is water for injection and the other solvent is an aqueous organic acid, preferably aqueous acetic acid.
32. The method of any of items 30-31, wherein step a) comprises mixing the stabilizing agent and the osmotic agent and optionally a pharmaceutically acceptable solvent, preferably aqueous acetic acid, to obtain the first composition.
32. The method of any of items 30-31, wherein step b) comprises mixing Cetrorelix or a pharmaceutically acceptable salt thereof with a pharmaceutically acceptable solvent, preferably aqueous acetic acid, to obtain the second composition.
33. The method of any of items 30-32, wherein the second composition is an aqueous solution of Cetrorelix or a pharmaceutically acceptable salt thereof.
34. The method of any of items 30-33, further comprising e) sterile filtration of the liquid composition obtained in step c) or d) to obtain a sterile liquid composition.
35. The method of any of items 30-34, wherein step c) further comprises adding a pharmaceutically acceptable solvent, preferably water for injection, to the first and second composition.
36. The method of any of items 30-35, further comprising the step of adjusting the pH of the liquid composition obtained in step c) or d) to a predetermined value by adding a pharmaceutically acceptable acid, preferably acetic acid, or a pharmaceutically acceptable base, preferably sodium hydroxide.
37. The method of item 36, wherein the predetermined pH value is 4.0-6.0, preferably 4.3-5.1, and more preferably 4.5-5.0.
38. The method of any of items 30-37, further comprising filling the liquid composition obtained in step c), d), or e) in a container, such as an ampoule, vial or an injection device, such as a syringe or an autoinjector.

### Figures

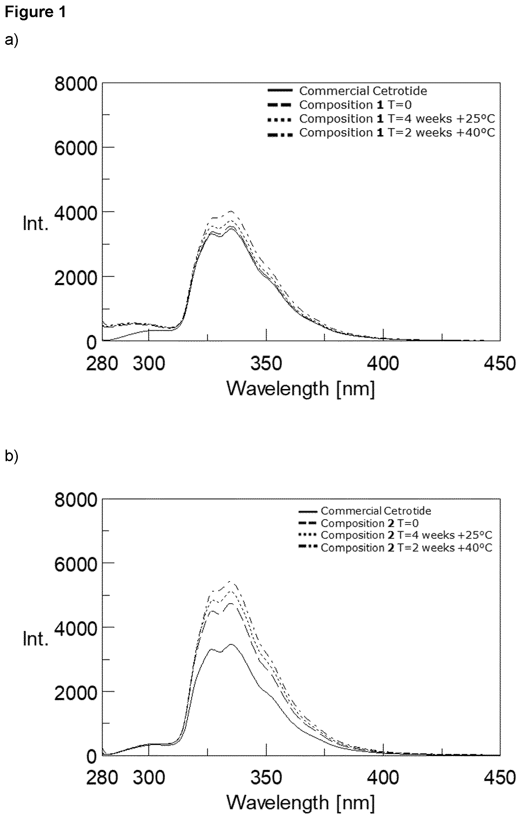

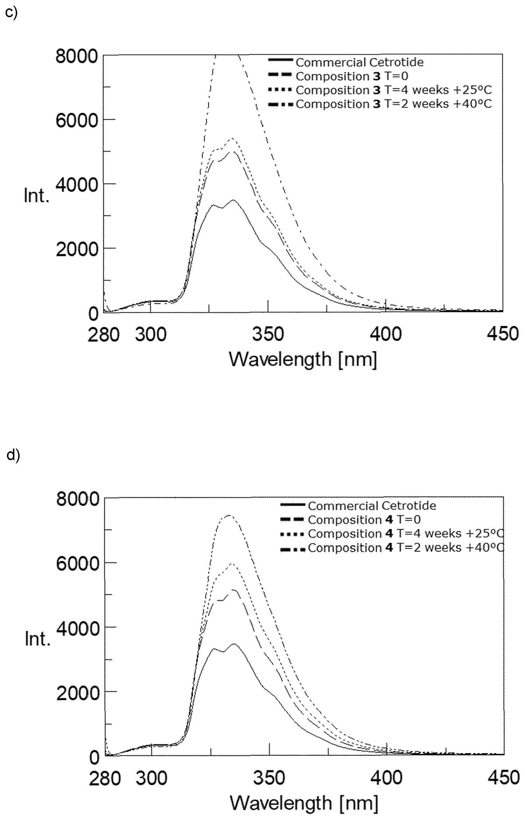

**Fig. 1** shows fluorescence spectra of liquid Cetrorelix compositions including (a, b) and lacking (c, d) a stabilizing agent. Spectrum "a" corresponds to Composition 1, spectrum "b" corresponds to Composition 2, spectrum "c" corresponds to Comparative Composition 3 and spectrum "d" corresponds to Comparative Composition 4. The spectrum at time zero is compared with the spectrum after storage for two weeks at 40°C, after storage for four weeks at 25°C, and the spectrum of commercially available Cetrotide^{®}.

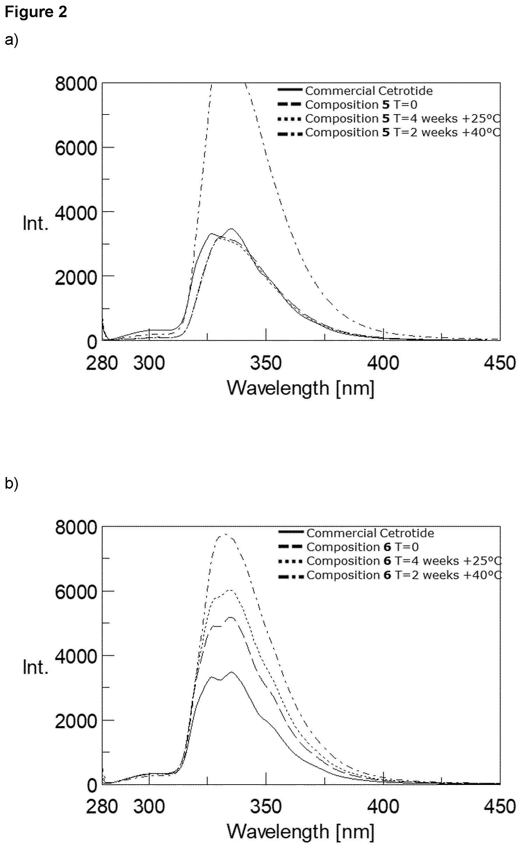

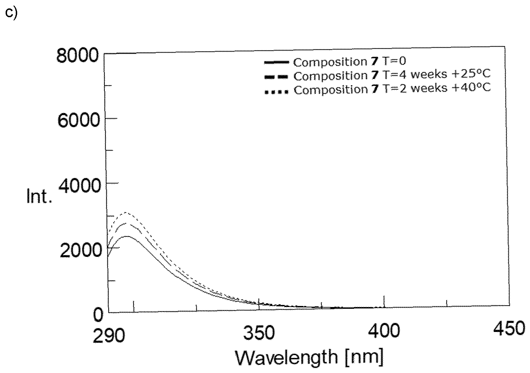

**Fig. 2** shows fluorescence spectra of comparative Cetrorelix compositions including arginine monohydrochloride (a), proline (b) and phenol (c) as a stabilizing agent. Spectrum "a" corresponds to Comparative Composition 5, spectrum "b" corresponds to Comparative Composition 6, and spectrum "c" corresponds to Comparative Composition 7. The spectrum at time zero is compared with the spectrum after storage for two weeks at 40°C , after storage for four weeks at 25°C, and the spectrum of commercially available Cetrotide^{®}.

The present invention is further described by, but not limited to, the following examples.

### Example 1

Four different Cetrorelix acetate compositions were prepared as follows.

Composition 1: Cetrorelix acetate was dissolved in a mixture of D-mannitol, benzyl alcohol and water for injection. Then, diluted acetic acid was added in an amount to adjust the pH of the composition to a value of 5.0. The amount of D-mannitol in the final composition was 37.80 mg/ml, the amount of benzyl alcohol in the final composition was 10.00 mg/ml and the amount of Cetrorelix acetate in the final composition was 0.25 mg/ml Cetrorelix pure peptide in its acetate salt form. The osmolality of the final composition was 316 mOsm/kg. The composition was sterile filtered and filled in a glass vial.

Composition 2: Cetrorelix acetate was dissolved in a mixture of D-mannitol, poloxamer 188 and water for injection. Then, acetic acid was added in an amount to adjust the pH of the composition to a value of 5.0. The amount of D-mannitol in the final composition was 55.00 mg/ml, the amount of poloxamer 188 in the final composition was 0.50 mg/ml and the amount of Cetrorelix acetate in the final composition was 0.25 mg/ml Cetrorelix pure peptide in its acetate salt form. The osmolality of the final composition was 325 mOsm/kg. The composition was sterile filtered and filled in a glass vial.

Comparative composition 3: Cetrorelix acetate was dissolved in a mixture of D-mannitol and water for injection. Then, acetic acid was added in an amount to adjust the pH of the composition to a value of 5.0. The amount of D-mannitol in the final composition was 55.00 mg/ml and the amount of Cetrorelix acetate in the final composition was 0.25 mg/ml Cetrorelix pure peptide in its acetate salt form. The osmolality of the final composition was 322 mOsm/kg. The composition was sterile filtered and filled in a glass vial.

Comparative composition 4: Cetrorelix acetate was dissolved in a mixture of D-mannitol and water for injection. Then, glutamic acid was added in an amount to adjust the pH of the composition to a value of 5.0. The amount of D-mannitol in the final composition was 55.00 mg/ml and the amount of Cetrorelix acetate in the final composition was 0.25 mg/ml Cetrorelix pure peptide in its acetate salt form. The osmolality of the final composition was 322 mOsm/kg. The composition was sterile filtered and filled in a glass vial.

The compositions were examined for their stability and pharmaceutical properties by the following methods.

### pH measurement

pH measurements were performed on a calibrated Seven Multi pH meter (Mettler Toledo), equipped with a microelectrode.

No pH changes were observed for the compositions after two weeks of storage at 40°C (RH 75%) and after four weeks of storage at 25°C (RH 60%).

### Osmolality

The osmolality was determined by measuring the freezing point of formulations, using a OSMOMAT 030-D (Gonotec).

### Visual inspection for particle formation

The visual inspections were carried out by two different operators, classifying the formulation as:
- FVP: if the formulation was deemed free of visible particles;
- +: if few particles were observed;
- ++: if there were particles;
- +++: if many particles/large particulate/precipitation/flakes were observed;
- H: Hurricane, tornado, e.g., because sedimented or floating particles were observed;
- Gel: if the formulation appeared gelled.

Visual inspection was performed directly after preparation of the compositions (time 0), after two weeks of storage at 40°C (RH 75%) and after four weeks of storage at 25°C (RH 60%).

### Sub-visible particle detection by Aura

An Aura instrument (Halo Labs) was used to count and characterize sub-visible particles. This instrument performs various photophysical microscopy measurements, enabling the characterization of the physical nature (count, size, and morphology) and the identity (protein / non-protein) of particulates within the sample.

Samples were loaded onto a proprietary filter plate and vacuum dried, leaving particles behind on the surface of the filter membrane.

Aura uses proprietary patented backgrounded membrane imaging (BMI) whereby a background image of the filter membrane is first taken and then subtracted from the sample image to increase particle resolution. In addition, fluorescence membrane microscopy (FMM) was used to identify particles stained with fluorescent dyes (Thioflavin T), enabling the differentiation between protein versus non-protein species within the sample.

Sub-visible particles were detected directly after preparation of the compositions (time 0), after two weeks of storage at 40°C (RH 75%) and after four weeks of storage at 25°C (RH 60%).

### Protein content via SoloVPE

A SoloVPE instrument (C Technologies) is a UV-Vis spectrophotometer that allows measuring the peptide content using the Beer-Lambert law. Measurements were performed directly after preparation of the compositions (time 0), after two weeks of storage at 40°C (RH 75%) and after four weeks of storage at 25°C (RH 60%).

### Turbidity by nephelometry

The clarity/turbidity of the samples was analyzed using Turbidimeter mod. 2100 ANIS (Hach Lange). The results are given in Nephelometric Turbidity Units (NTU).

A liquid is considered clear (limpid) if its brightness or clarity is ≤3 NTU. More in detail:
- a liquid is considered clear if its opalescence is ≤ 3 NTU;
- a liquid is considered slightly opalescent if its opalescence is > 3 NTU but ≤ 6 NTU;
- a liquid is considered opalescent if its opalescence is > 6 NTU but ≤ 18 NTU; and
- a liquid is considered highly opalescent if its opalescence is > 18 NTU but ≤ 30 NTU.

### Stability via intrinsic fluorescence analysis

The intrinsic fluorescence analyzed via a spectrofluorometer (Uncle, Unchained Labs) was used to monitor possible changes in the stability of the compositions: the fluorescence spectrum of each formulation was compared with the Cetrotide^{®} freeze-dry fluorescence spectrum, and then also after stressed and accelerated storage conditions the fluorescent profile was monitored.

### Results

### a) Particle formation

The Cetrorelix compositions were examined for the presence of visible and sub-visible particles by visual inspection and Aura, respectively.

**Table 1. Visual inspection of compositions 1-4**

| **Composition** | **time 0** | **2 weeks (40°C)** | **4 weeks (25°C)** |
|---|---|---|---|
| 1 | + | FVP | FVP |
| 2 | FVP | FVP | FVP |
| 3 | ++ | ++ | ++ |
| 4 | ++ | ++ | ++ |

While compositions including a stabilizer according to the present invention showed no or only few particles after preparation and storage, comparative compositions 3 and 4 included a clearly visible amount of particles.

Sub-visible particles detected through BMI (all particles per ml present in the sample) are reported in Table 2, while proteinaceous particles detected by FMM are reported in Table 3. Compositions 1-3 contained comparative amounts of sub-visible (proteinaceous) particles.

**Table 2. Sub-visible particles by Aura (BMI)**

| **Composition** | **time 0** | | **2 weeks (40°C)** | | **4 weeks (25°C)** | |
|---|---|---|---|---|---|---|
| | **SVP > 5µm** | **SVP > 25µm** | **SVP > 5µm** | **SVP > 25µm** | **SVP > 5µm** | **SVP > 25µm** |
| 1 | 2880 | 120 | 2120 | 40 | 2520 | 120 |
| 2 | 1040 | 80 | 2360 | 240 | 5680 | 240 |
| 3 | 1200 | 120 | 2600 | 120 | 1720 | 40 |
| 4 | 16960 | 200 | 4640 | 40 | 6440 | 320 |

**Table 3. Sub-visible proteinaceous particles**

| **Composition** | **time 0** | | **2 weeks (40°C)** | | **4 weeks (25°C)** | |
|---|---|---|---|---|---|---|
| | **SVP > 5µm** | **SVP > 25µm** | **SVP > 5µm** | **SVP > 25µm** | **SVP > 5µm** | **SVP > 25µm** |
| 1 | 1720 | 120 | 1160 | 40 | 1960 | 120 |
| 2 | 920 | 80 | 2080 | 240 | 3880 | 160 |
| 3 | 1040 | 80 | 1160 | 80 | 1520 | 0 |
| 4 | 16760 | 200 | 1720 | 0 | 6120 | 320 |

### b) Peptide content

The peptide content was monitored at time zero, after two weeks of storage at 40°C (RH 75%) and after four weeks of storage at 25°C (RH 60%) by SoloVPE. No peptide loss was seen in any of these conditions for compositions 1-4.

### c) Turbidity

The turbidity of the compositions was examined at time zero, after two weeks of storage at 40°C (RH 75%) and after four weeks of storage at 25°C (RH 60%) by nephelometry. The results are shown in table 4.

**Table 4. NTU values obtained for compositions 1-4**

| **Composition** | **time 0** | **2 weeks (40°C)** | **4 weeks (25°C)** |
|---|---|---|---|
| 1 | 2 | 2 | 3 |
| 2 | 3 | 2 | 2 |
| 3 | 4 | 4 | 3 |
| 4 | 3 | 4 | 2 |

Clear formulations (≤ 3 NTU) were obtained for the compositions according to the invention comprising a stabilizing agent. The comparative compositions were slightly opalescent.

### d) Stability

The stability of the compositions was evaluated via intrinsic fluorescence at time zero, after two weeks of storage 40°C (RH 75%) and after four weeks of storage at 25°C (RH 60%).

As it can be seen in Figure 1, only formulations containing a stabilizing agent were able to maintain the same fluorescent peptide profile after storage, i.e. showed good stability (Fig. 1a and 1b). The comparative compositions showed altered fluorescence intensities after storage, indicating peptide aggregation (Fig. 1c and 1d).

### Example 2

Five different Cetrorelix acetate compositions including a stabilizing agent were examined. Besides composition 1 (including benzyl alcohol) and composition 2 (including poloxamer 188) according to the invention as described in Example 1, the following comparative compositions were manufactured.

Comparative composition 5: Cetrorelix acetate was dissolved in a mixture of D-mannitol, arginine monohydrochloride and water for injection. Then, diluted acetic acid was added in an amount to adjust the pH of the composition to a value of 5.0. The amount of D-mannitol in the final composition was 49.19 mg/ml, the amount of arginine monohydrochloride in the final composition was 3.16 mg/ml and the amount of Cetrorelix acetate in the final composition was 0.25 mg/ml Cetrorelix pure peptide in its acetate salt form. The osmolality of the final composition was 325 mOsm/kg. The composition was sterile filtered and filled in a glass vial.

Comparative composition 6: Cetrorelix acetate was dissolved in a mixture of D-mannitol, proline and water for injection. Then, acetic acid was added in an amount to adjust the pH of the composition to a value of 5.0. The amount of D-mannitol in the final composition was 49.19 mg/ml, the amount of proline in the final composition was 3.45 mg/ml and the amount of Cetrorelix acetate in the final composition was 0.25 mg/ml Cetrorelix pure peptide in its acetate salt form. The osmolality of the final composition was 322 mOsm/kg. The composition was sterile filtered and filled in a glass vial.

Comparative composition 7: Cetrorelix acetate was dissolved in a mixture of D-mannitol, phenol and water for injection. Then, acetic acid was added in an amount to adjust the pH of the composition to a value of 5.0. The amount of D-mannitol in the final composition was 35.30 mg/ml, the amount of phenol in the final composition was 10.00 mg/ml and the amount of Cetrorelix acetate in the final composition was 0.25 mg/ml Cetrorelix pure peptide in its acetate salt form. The osmolality of the final composition was 318 mOsm/kg. The composition was sterile filtered and filled in a glass vial.

Compositions 1, 2, and 5-7 were analyzed as set forth in Example 1.

### Results

### a) Particle formation

The Cetrorelix compositions were examined for the presence of visible and sub-visible particles by visual inspection and Aura, respectively.

**Table 5. Visual inspection of compositions 1-2 and 5-7**

| **Composition** | **time 0** | **2 weeks (40°C)** | **4 weeks (25°C)** |
|---|---|---|---|
| 1 | + | FVP | FVP |
| 2 | FVP | FVP | FVP |
| 5 | ++ | ++ | ++ |
| 6 | ++ | ++ | ++ |
| 7 | + | + | FVP |

While compositions including a stabilizer according to the present invention showed no or only few particles after preparation and storage, comparative compositions 5 and 6 comprising amino acid stabilizers included a clearly visible amount of particles.

Sub-visible particles detected through BMI (all particles per ml present in the sample) are reported in Table 6, while proteinaceous particles detected by FMM are reported in Table 7. It was found that the presence of proline and phenol stabilizing agents (comparative compositions 6 and 7) lead to a significantly increased amount of sub-visible particles in the liquid Cetrorelix composition.

**Table 6. Sub-visible particles by Aura (BMI)**

| **Composition** | **time 0** | | **2 weeks (40°C)** | | **4 weeks (25°C)** | |
|---|---|---|---|---|---|---|
| | **SVP > 5µm** | **SVP > 25µm** | **SVP > 5µm** | **SVP > 25µm** | **SVP > 5µm** | **SVP > 25µm** |
| 1 | 2880 | 120 | 2120 | 40 | 2520 | 120 |
| 2 | 1040 | 80 | 2360 | 240 | 5680 | 240 |
| 5 | 1760 | 80 | 720 | 0 | 1840 | 160 |
| 6 | 7120 | 120 | 1280 | 120 | 32920 | 440 |
| 7 | 4280 | 480 | 9000 | 160 | 5640 | 440 |

**Table 7. Sub-visible proteinaceous particles**

| **Composition** | **time 0** | | **2 weeks (40°C)** | | **4 weeks (25°C)** | |
|---|---|---|---|---|---|---|
| | **SVP > 5µm** | **SVP > 25µm** | **SVP > 5µm** | **SVP > 25µm** | **SVP > 5µm** | **SVP > 25µm** |
| 1 | 1720 | 120 | 1160 | 40 | 1960 | 120 |
| 2 | 920 | 80 | 2080 | 240 | 3880 | 160 |
| 5 | 1600 | 80 | 480 | 0 | 1140 | 120 |
| 6 | 6880 | 80 | 960 | 80 | 32280 | 400 |
| 7 | 1480 | 240 | 5240 | 160 | 3360 | 440 |

### b) Peptide content

The peptide content was monitored at time zero, after two weeks of storage at 40°C (RH 75%) and after four weeks of storage at 25°C (RH 60%) by SoloVPE. Due to significant interference from phenol in the SoloVPE measurement, no result are available for composition 7.

**Table 8. Peptide content detected by SoloVPE**

| **Composition** | **time 0** | **2 weeks (40°C)** | **4 weeks (25°C)** |
|---|---|---|---|
| 1 | 0.25 | 0.25 | 0.25 |
| 2 | 0.25 | 0.25 | 0.25 |
| 5 | 0.03 | 0.03 | 0.09 |
| 6 | 0.23 | 0.27 | 0.26 |

The presence of arginine stabilizing agent (comparative composition 5) lead to a severe loss in peptide, while no peptide loss was seen in any conditions for compositions 1, 2 and 6.

### c) Turbidity

The turbidity of the compositions was examined at time zero, after two weeks of storage at 40°C (RH 75%) and after four weeks of storage at 25°C (RH 60%) by nephelometry. To evaluate the impact of shaking stress on peptide stability, all formulations were additionally investigated after being subjected to a shaking stress of 300 rpm for 24 hours at room temperature. The results are shown in table 9.

**Table 9. NTU values obtained for compositions 1-2 and 5-7**

| **Composition** | **time 0** | **2 weeks (40°C)** | **4 weeks (25°C)** | **shaking stress** |
|---|---|---|---|---|
| 1 | 2 | 2 | 3 | 2 |
| 2 | 3 | 2 | 2 | 2 |
| 5 | 4 | 2 | 3 | 5 |
| 6 | 3 | 4 | 3 | 3 |
| 7 | 6 | 2 | 3 | 48 |

Clear formulations (≤ 3 NTU) were obtained for compositions 1 and 2 according to the invention. The comparative compositions were slightly opalescent.

Shaking stress did not have a significant impact on compositions 1-2 and 5-6, while the phenol-stabilized composition 7 was very opalescent and particle formation could be detected after shaking.

### d) Stability

The stability of the compositions was evaluated via intrinsic fluorescence at time zero, after two weeks of storage 40°C (RH 75%) and after four weeks of storage at 25°C (RH 60%). For compositions 1-6 the fluorescence was analyzed using an emission wavelength of 275nm. Due to interference caused by the adsorption of phenol in the same area as Cetrorelix, composition 7 was analyzed with an emission wavelength of 285nm.

Formulations containing a stabilizing agent according to the invention were able to maintain the same fluorescent peptide profile after storage, i.e. showed good stability (Fig. 1a and 1b). The comparative compositions showed altered fluorescence intensities after storage, indicating peptide aggregation (Fig. 2a and 2b).

### Example 3

Computational investigations were also performed on Cetrorelix.

### Methods

The initial structure of Cetrorelix was built in Molecular Operating Environment (MOE) 2020 software with default Amber10:EHT force field. The MD simulations were performed using NAMD software using CHARMM36m force field and TIP3P model for water. Nonbonded interactions were calculated with a cutoff of 12 Å, with a switching function starting at 10 Å. Particle mesh Ewald (PME) was used to account for long-range electrostatic interactions with a grid density of 1 Å-3 and a PME interpolation order of 6. Rigid bonds for all hydrogen atoms were enforced using the SHAKE algorithm. All simulations used for analysis were performed using periodic boundary conditions in the NPT ensemble. The structure of poloxamer was constructed using an in-house script that considers a total molecular weight of 8400 g/mol and a building block composition of 80% / 20% of polyoxyethylene / polyoxypropylene. The equilibration protocol of each system consisted in a temperature ramp from 0 K to 300 K, the latter chosen as the target temperature for all systems in production runs. The simulation box contained either 1 single Cetrorelix molecule or an ensemble of 4 Cetrorelix molecules separated by at least 10 Å from each other. Each system was simulated for 300 ns and was replicated three-folds. Analysis of the trajectories was performed in VMD using custom scripts.

### Results

In a first computational investigation, Cetrorelix was simulated in the presence of either poloxamer 188 or benzyl alcohol. The results suggest that the mechanism of stabilization of both excipients is tightly bound to their interaction with hydrophobic regions of Cetrorelix. By preventing the Cetrorelix' exposure to the aqueous environment, both poloxamer 188 and benzyl alcohol effectively reduce the propensity for self-interaction between peptide molecules, thereby lowering the risk of aggregation.

In a second computational investigation, Cetrorelix was simulated in the presence of either lactic acid or acetic acid. The results suggest that the negatively charged conjugated base from the buffer strongly interacts with positively charged residues in Cetrorelix, leading to increased aggregation between Cetrorelix molecules. Besides through the neutralization of the positive charge, aggregation is further facilitated by buffer coordination effect of multiple residues from different Cetrorelix molecules. To minimize the quantity of conjugated base in the formulation, the use of free acid to fix the pH is preferred over buffer solutions. Furthermore, buffer agents with lower pKa values may reduce the risk of aggregation, as they can be used to reach a lower pH value with a relatively lower amount of acid.

## Claims

1. A liquid pharmaceutical composition, comprising and preferably consisting of:
- Cetrorelix or a pharmaceutically acceptable salt thereof, preferably Cetrorelix acetate,
- a stabilizing agent selected from poloxamer having an average molecular weight of 7,000-12,000 g/mol, benzyl alcohol, or a mixture thereof,
- an osmotic agent, and
- a pharmaceutically acceptable solvent comprising an organic acid.

2. The liquid pharmaceutical composition according to claim 1, wherein the stabilizing agent is poloxamer 188, preferably comprised in an amount of 0.5-10.0 mg/ml, more preferably 1.0-5.0 mg/ml, or benzyl alcohol, preferably comprised in an amount of 5.0-20.0 mg/ml, more preferably 7.5-15.0 mg/ml.

3. The liquid pharmaceutical composition according to any of the preceding claims, wherein the osmotic agent is selected from the group consisting of mannitol, glycerol, sorbitol, sodium chloride, potassium chloride, dextrose, sucrose, trehalose, or a combination thereof, preferably D-mannitol.

4. The liquid pharmaceutical composition according to any of the preceding claims, wherein the organic acid is acetic acid, lactic acid, glutamic acid, gluconic acid, succinic acid, or a mixture thereof, preferably acetic acid, preferably comprised in a concentration of 0.05-0.50 mg/ml, more preferably 0.10-0.30 mg/ml.

5. The liquid pharmaceutical composition according to any of the preceding claims, wherein the pharmaceutically acceptable solvent is aqueous acetic acid.

6. The liquid pharmaceutical composition according to any of the preceding claims, wherein the pH of the pharmaceutical composition is 4.0-6.0, preferably 4.3-5.1, and more preferably 4.5-5.0.

7. The liquid pharmaceutical composition according to any of the preceding claims, wherein the pharmaceutical composition comprises and preferably consists of Cetrorelix acetate in a concentration of 0.2 -0.3 mg/ml Cetrorelix pure peptide in its acetate salt form, poloxamer 188 in a concentration of 1.5 mg/ml, D-mannitol in a concentration of 54.8 mg/ml, acetic acid in a concentration of 0.1 mg/ml, and water for injection.

8. The liquid pharmaceutical composition according to any of the preceding claims, wherein the pharmaceutical composition comprises and preferably consists of Cetrorelix acetate in a concentration of 0.2 - 0.3 mg/ml Cetrorelix pure peptide in its acetate salt form, benzyl alcohol in a concentration of 10 mg/ml, D-mannitol in a concentration of 37.8 mg/ml, acetic acid in a concentration of 0.1 mg/ml, and water for injection.

9. The liquid pharmaceutical composition according to any of the preceding claims, which is a single-dose formulation, or a multi-dose formulation comprising a pharmaceutically acceptable preservative in an effective amount.

10. The liquid pharmaceutical composition according to any of the preceding claims, which is in the form of a solution, preferably for parenteral administration.

11. A pharmaceutical dosage form comprising the liquid pharmaceutical composition according to any of claims 1-10 in a container, such as an ampoule or vial, preferably a sterile vial, or an injection device, preferably a pre-filled syringe or an autoinjector.

12. A liquid pharmaceutical composition according to any of claims 1-10 or a pharmaceutical dosage form according to claim 11 for use in inhibiting premature luteinizing hormone surges in woman undergoing controlled ovarian stimulation.

13. A method for preparing a liquid pharmaceutical composition according to any of claims 1-10 or a pharmaceutical dosage form according to claim 11, comprising the steps of:
a) providing a first composition comprising a stabilizing agent selected from poloxamer having an average molecular weight of 7,000-12,000 g/mol, benzyl alcohol, or a mixture thereof, and an osmotic agent, preferably D-mannitol;
b) providing a second composition comprising or consisting of Cetrorelix or a pharmaceutically acceptable salt thereof, preferably Cetrorelix acetate;
c) mixing the first composition of step a) and the second composition of step b) to obtain a liquid composition; and
d) optionally adding an organic acid to the mixture obtained in step c),
wherein a pharmaceutically acceptable solvent is present in the first composition and/or wherein a pharmaceutically acceptable solvent is present in the second composition.

14. The method of claim 13, wherein a pharmaceutically acceptable solvent is present in the first and second composition, wherein the solvent of the first and second composition is the same or different, preferably wherein one of the solvents is water for injection and the other solvent is aqueous acetic acid.

15. The method of any of claims 13-14, further comprising the step of adjusting the pH of the liquid composition obtained in step c) to a predetermined value by adding a pharmaceutically acceptable acid, preferably acetic acid, or a pharmaceutically acceptable base, preferably sodium hydroxide, wherein the predetermined pH value is preferably 4.0-6.0, more preferably 4.3-5.1, and particularly 4.5-5.0.
